# EUROPEAN PATENT SPECIFICATION

(11) **EP 1 846 562 B1**
(45) Date of publication and mention of the grant of the patent: **14.07.2010**
(21) Application number: 06716589.4
(22) Date of filing: 26.01.2006
(51) Int. Cl.: C12N 15/85, A01K 61/00

(54) **MEANS AND METHODS FOR IMPROVING THE DEVELOPMENT AND MATURATION OF EGGS AND/OR SPERM IN FISH USING HORMONES PRODUCED BY TRANSPLANTED CELLS**
MITTEL UND METHODEN UM DIE ENTWICKLUNG UND REIFUNG VON FISCH EIZELLEN ODER SPERMIEN ZU FÖRDERN DURCH VERABREICHUNG VON DURCH TRANSPLANTIERTEN ZELLEN PRODUZIERTEN HORMONEN
MOYENS ET MÉTHODES POUR AMÉLIORER LE DÉVELOPEMENT ET LA MATURATION D'OOCYTES OU DE SPERMES DE POISSON UTILISANT DES HORMONES PRODUITES PAR CES CELLULES TRANSPLANTÉES.

(30) Priority: 26.01.2005 EP 05075204; 28.02.2005 EP 05075490
(43) Date of publication of application: 24.10.2007
(73) Proprietor: Universiteit Leiden, 2311 EZ Leiden (NL)
(72) Inventor: SPAINK, Herman Pieter, NL-2341 SG Oegstgeest (NL); VAN DEN THILLART, Guido Everard Elisabeth Johannes, NL-2353 EA Leiderdorp (NL); SCHNABEL PERAZA, Denhi, NL-2313 MJ Leiden (NL)
(74) Representative: Hatzmann, Martin
(86) International application number: PCT/NL2006/000043
(87) International publication number: WO 2006/080841

(56) References cited:
- WO-A-02/081680
- US-A- 4 647 552
- MORITA TETSURO ET AL: "Fish eggs as bioreactors: the production of bioactive luteinizing hormone in transgenic trout embryos" TRANSGENIC RESEARCH, vol. 13, no. 6, December 2004 (2004-12), pages 551-557, XP002344788 ISSN: 0962-8819
- PEDERSEN BENEDIKTE HEDEGAARD: "Fertilisation of eggs, rate of embryonic development and hatching following induced maturation of the European eel Anguilla anguilla" AQUACULTURE, vol. 237, no. 1-4, 2 August 2004 (2004-08-02), pages 461-473, XP002344789 ISSN: 0044-8486
- LEONARDO A F G ET AL: "Induced spawning of hatchery-raised Brazilian catfish, cachara Pseudoplatystoma fasciatum (Linnaeus, 1766)" AQUACULTURE, ELSEVIER, vol. 240, no. 1-4, 27 October 2004 (2004-10-27), pages 451-461, XP004587181 ISSN: 0044-8486
- GARCIA-ALONSO J ET AL: "Induction of oocyte maturation in the white croaker Micropogonias furnieri (Pisces: Sciaenidae) by human chorionic gonadotropin." BRAZILIAN JOURNAL OF BIOLOGY, vol. 64, no. 1, February 2004 (2004-02), pages 73-80, XP002344791 ISSN: 1519-6984
- STOKSTAD ERIK: "Transgenic species. Engineered fish: friend or foe of the environment?" SCIENCE. 13 SEP 2002, vol. 297, no. 5588, 13 September 2002 (2002-09-13), pages 1797-1799, XP002344790 ISSN: 1095-9203

## Description

The invention relates to the field of fish culture. The invention in particular relates to the field of hormone driven improvement of the development and maturation of eggs and/or sperm in fish.

Many fish species mature in response to environmental factors. These factors such as light cycle, temperature, season, pressure, and energy reserves, are sensed by the animal and control the inhibitory action of hypothalamic centres on the pituitary. In this way the production of gonadotropins by the pituitary is normally depressed and activated only under certain environmental conditions. When activated, the pituitary releases gonadotropins that stimulate the growth and development of both male and female gonads.

For fisheries it is important that maturation can typically be stimulated artificially by regular injections with the same hormones, which consist often of crude pituitary extracts. The regular injections overrule to some extend the environmental triggers for the development of both the male and female gonads. However, the current practice of artificially improving the development and/or maturation of eggs and/or sperm using hormones is not completely satisfactory. For instance, in eels, which are still immature when they commence their spawning migration, the females have to be treated weekly during 3-5 months before oocytes are ripe enough for ovulation. This is a time consuming procedure and stressful for the fish.

The present invention provides a method for improving the development and/or maturation of eggs and/or sperm in fish using fertility hormone administration comprising providing said fish with cells producing said fertility hormone for use in the creation of progeny or the production of eggs. The cells that are transplanted into the fish release one or more hormones, thereby at least reducing the need for regular injections with the hormone(s) themselves. The cells may be transplanted in various ways as long as the secreted hormone(s) are released into the circulation system and reach all tissues and in particular the sexual organs in sufficient quantity. The cells may be transplanted anywhere in the body. Implants, consisting of hormone(s) producing cells, are typically used to bypass the pituitary gland in order to produce fertility hormones such as luteinizing hormone (LH), follicle stimulating / hormone (FSH) or chorionic gonadotropin (CG). The implants with hormone producing cells are preferably inserted into sites that have access to the bloodstream. Preferred methods of insertion are intra-peritoneal, and subcutaneous injection. Sites with access to the bloodstream are very well suited for cells that produce secondary hormones such as LH, FSH, and CG. Transplantation of cells is often used in mammals and a lot of experience has been obtained with respect to methods to transplant and maintain cells for at least some time. Considering that the fish are typically killed after spawning or harvesting of the ripened eggs or sperm, there is no great need for control over the transplanted cells. Important is that the cells remain present in sufficient numbers to allow complete development and maturation of the gonads. Thus the number of cells may not be less than required for development and maturation and the cells may not be (or become) so numerous that they impede the development and maturation of the gonads or the general health of the animal. The level of production of the hormone by the cells is not very critical. The number of injected cells will depend on the total quantity required for the maturation. The hormone production will be quantified by a bio assay. The hormone release of the injected cells needs to be sufficiently high to stimulate the development and/or maturation of eggs and/or sperm. The upper boundary for expression of the hormone is not critical as over-expression of a fertility hormone is not toxic in itself and does not negatively affect the stimulation of maturation and/or development of eggs and/or sperm. In the mammalian world, several types of grafting aids have been developed to allow for prolonged stay of the cells or to allow differentiation of the cells. These aids can of course also be used in the present invention. Such aids include, but are not limited to, collagen or synthetic matrices for the grafting and attachment of cells.

As the cells, in many cases, need not be present for a very long time, it is possible to transplant fish cells from many different species into a recipient fish. If the evolutionary difference between the transplanted cells and the recipient is large, it is likely that the recipient fish will mount an immune response to the transplanted cells (the graft). However, as the cells often need only be present for a limited amount time, such immune response can typically be tolerated. To increase the robustness and predictability of the procedure it is preferred that the graft is derived from the same genus or family as the recipient fish species. Preferably, the two are from the same species. As fish are typically outbred populations there are immunological differences between fish of the same species. This is typically not a problem as shown in one of our examples of experiments with eels, however, it is possible to further match the graft and the recipient for common immunological markers. Typical markers are major and minor histocompatibility antigens. The grafting of the transplanted cells may further be facilitated by providing the recipient fish with immunosuppressants such as cyclosporin.

The transplantation of hormone producing cells of the invention can be used for improving the maturation of eggs and/or sperm, the fertility of the eggs and/or sperm, the insemination of eggs, the quality of the resulting embryos, the survival of fertilized and unfertilised eggs and the survival of embryos. These improvements all lead to improved development and maturation of eggs and/or sperm in fish. The term development and maturation of eggs and/or sperm in fish is therefore not limited to the natural process of spawning but also relates to artificial methods for egg insemination. Thus, it also relates to the harvesting of unfertilised eggs and/or sperm from fish treated with a method of the invention. The unfertilised eggs may also be used for other purposes than the creation of progeny. A non-limiting example thereof is the production of eggs for human consumption such as caviar.

The development and maturation of eggs and/or sperm in fish can be stimulated in various ways. In one embodiment of the present invention the development and maturation of eggs and/or sperm in fish is said to be stimulated when the absolute number or the quality of the eggs, sperm or embryo's resulting from fertilized eggs is increased.

Reproduction is a highly regulated biological process. Different aspects of reproduction are regulated by different hormones. However several hormones can produce more or less similar effects when expressed by a cell that is transplanted into a fish. These hormones include: FSH, LH, CG (Chorionic gonadotropin), or a combination thereof. The hormones mentioned above are also known under different names. As the underlying amino acid sequence is the same, the hormones referred to by the synonyms are also within the scope of the invention. For instance, FSH is also referred to as Follicle stimulating hormone, follitropin or gametocinetic hormone. LH is also referred to as Luteinizing hormone, Luteotropin or interstitial cell stimulating hormone (ICSH). CG (Chorionic gonadotropin) is also referred to as Chorionic gonadotropic hormone, chorionic gonadotropic hormone, choriogonadotropin or chorionic gonadotropin.

In a preferred embodiment of the invention said hormone is a hormone directly involved in the development and maturation of eggs. Such fertility hormones are typically produced by the pituitary, or the sexual organs. In a preferred embodiment the fertility hormone comprises luteinizing hormone (LH), follicle stimulating hormone (FSH), or chorionic gonadotropin (CG) or a functional part, derivative and/or analogue of such a hormone. These hormones are very portent stimulators of the development and maturation of eggs and/or sperm in fish. These hormones are very conserved in nature and hardly have a species barrier. For instance, the presence of human fertility hormones in urine can be detected by incubating them with frog eggs. Similarly, human chorionic gonadotropin (hCG) also works on eel and carp and salmon pituitary extracts work on many different fish species such as eel, seabream and trout.

It is possible that the recipient develops an immune response against a heterologous hormone. Although this immune response is typically too late to affect the stimulation of maturation and/or development of eggs and/or sperm, it is preferred that the hormone is a fish hormone or a functional part, derivative and/or analogue thereof. This limits the divergence between the provided and the endogenous hormone, thereby at least in part limiting the development of an immune response against the provided hormone in the recipient. Preferably, the hormone is derived from a species that belongs to the same genus as the recipient. In this way the chance that an immune response is developed is further reduced. In a particularly preferred embodiment a provided hormone is immunologically identical to the equivalent thereof in the recipient. This completely prevents the development of any detrimental immune response against the provided hormone.

The cells can either express the desired hormone without manipulation or can be manipulated to express the desired hormone. When the cells do not express the hormone already or do not express sufficient hormone, they can be provided with the genetic information for expressing the hormone.

In a preferred embodiment the cells are provided with the genetic information to express the fertility hormone. This can be done by providing the cells with expression cassettes comprising coding sequences for the hormones. However, it is also possible to activate the endogenous genes by inserting an active regulatory sequence near the coding sequence(s) for the respective hormones. This can be done for instance through homologous recombination.

The LH and FSH proteins belong to a family of related proteins. Both share the characteristic that they are functional as heterodimers consisting of a common α-subunit and a different β-subunit. In the case of hormones that consist of more than one protein chain it is possible that cells do not express all of the chains needed to generate the hormone. In these cases only expression cassettes are required for the chain(s) that are lacking. Thus, if one or more but not all of the subunits of the hormone are adequately expressed in the cells one only needs to express the remaining subunit(s) in the cell. If none of the subunits are expressed, one has to manipulate the cells such that all of the subunits are expressed at adequate levels. In a preferred embodiment, the cells are provided with expression cassettes for the subunits of the hormone. In a preferred embodiment the cells are provided with expression cassettes for the three protein chains that make up LH and FSH (i.e. for the common α-subunit and the unique β-subunits for each of the hormones), or in separate cell lines the combination of βLH + α and βFSH + α are expressed. Thus in a preferred embodiment said cells are genetically modified to express said hormone(s). Preferably, the cells are provided with one or more genes encoding said hormone(s).

The cells can be primary cells or cell lines that are cultured in vitro for an extended period. In a preferred embodiment, the cells are derived from a clonal population of cells. In this way, the cells can be subjected to detailed quality control prior to use. This also allows for the generation of cell banks that have the same property. Moreover, a clonal population can be subjected to further manipulations. For instance, if one wants to reduce immune responses in the recipient, it is possible to knock out expression of major and/or minor histocompatibility antigens. Thus in a preferred embodiment, the cells have been selected for a reduced immunogenicity in the recipient. A cell for use in a method or use of the invention can be a primary cell or a cultured cell. Preferably, said cell is a cultured cell, more preferably an immortalized cultured cell. Cultured cell are typically cell lines. Cell lines can be propagated for at least 5 passages without substantial change in the phenotype of said cell. Immortalized cell lines can be passaged at least 50 times without undergoing such phenotypic change. Immortalized cells can be obtained from primary cells in various ways. Preferably, said immortalized cell is obtained from a culture of primary cells that has undergone the crisis that is typically for primary cells in culture. In another preferred embodiment said cell has become immortalized through introduction of one or more genes into a primary cell.

A method of the invention may be used for all types of fish. Preferred fish are eel, seabass, seabream, halibut, salmon, trout, cod, carp, catfish, and sturgeon. However, the invention is particularly suited for diadromous and preferably semelparous fish. These fish take a long time before spawning and typically do not all respond similarly to outside signals. With a method of the invention it is possible to stimulate the development and maturation of the fish at least in part independently of the environmental stimuli. This introduces a large amount of predictability towards the starting point for the fish culture. In diadromous fish it is further possible to synchronize egg development and maturation such that work can be better scheduled in the production process.

The invention further provides an isolated and/or recombinant fish cell that produces a fertility hormone. In a preferred embodiment said cell is genetically modified to express said fertility hormone. The invention further provides a fish cell provided with the capacity to express a fertility hormone. Preferably, the fish cell is provided with a recombinant and/or isolated nucleic acid sequence encoding said fertility hormone. If the hormone consists of one or more subunits, the fish cell is preferably provided with an isolated and/or recombinant nucleic acid sequence encoding at least one subunit of said hormone. Preferably, the fish cell is provided with nucleic acid sequence encoding all subunits of said hormone. Preferably said cell is a cell of a consumer fish. In a preferred embodiment said cell originates from eel, seabass, seabream, halibut, salmon, trout, cod, carp, catfish or a sturgeon. Preferably said cell is a cell as described above, i.e. a primary cell or a cell derived from a cell line that is cultured in vitro for an extended period. In a preferred embodiment, the cells are derived from a clonal population of cells. Preferably said cell is a cultured cell, more preferably an immortalized cultured cell.

In a preferred embodiment use is made of implants of cells that can be cultured. Furthermore these cells are preferably clonal, and preferably selectable for characteristics. It is however also possible to make use of primary cultures, tissue, fertilized eggs, or embryonic material. A method for making transgenic fish eggs has been published (Morita et al 2004, Transgenic Research 13, 551). However, the mentioned transgenic fish eggs cannot be further propagated. Furthermore the expression of the introduced gene(s), as described by Morita et al, is not stable. Selection of the introduced genes in eggs is difficult and using fish eggs as biorectors is rather time consuming in relation to culturable cells because micro-injection for each of the eggs has to be used, whereas in cell-cultures we can make use of standard transfection technology. Culturable cells can have the advantage that are easily be stored, and made available at any time. Furthermore there are no ethical problems with working with culturable cells as compared to genetically modified eggs. Injection of a suspension of cultured cells is rather easy as compared to implantation of eggs, and/or embryos.

The invention further provides a fish that comprises a cell according to the invention. Preferably, said fish is a consumer fish. Preferably, said fish is a consumer juvenile or adult fish. The time to reproduction is preferably short. Primary embryonic material has the disadvantage that it might not be available or, not available in sufficient quantities. In a preferred embodiment said fish is an eel, seabass, seabream, halibut, salmon, trout, cod, carp, catfish or a sturgeon. In a preferred embodiment said fish comprises non-genetically modified sperm cells, oocytes and/or progenitors thereof.

### Examples

### Cloning of LHβ, FSHβ and a zebrafish genes

The genes of zebrafish are already published LHβ (AY424304), FSHβ (AY424303) and α (AY424306), also the genes of eel are reported for LHβ (AB175835) in *Anguilla japonica;* FSHβ (AY169722) in *Anguilla anguilla* and for α (AB175834) in *Anguilla japonica.* In order to clone LHβ, FSHβ and α primers were designed based on the cDNA sequence of each one:

The sequence used for the design of the primers of the LHβ was:

atatataaat ctggacacgc agagacactt acaacagcct gctgagcaac cgcaacgcct
gtcaagatgt tattggctgg aaatggtgtc ttctttctct tctctttgtt tttcctgctg
gcggctgctc agagcttggt ttttccacgc tgtgagctag taaatgagac ggtatcggtg
gaaaaagagg gctgtccaaa atgcctggtg tttcagacca ccatctgcag cggccactgc

gtaacaaggg atcccgttta caagagcccg ttttccaccg tccaccagac agtgtgcatg
taccgggacg tccgctatga gaccattaac ctgcccgact gttccgccgg cgtggacccg
cagatcacat acccggtggc gctgagctgc gactgcagtc tgtgcaccat aaacacttcc
gactgcacca tccagagcct gcagcccgac ttctgcatgt cccagagaga ggatttcccc
gcatactaga cctcgggcaa ctcacgtcaa cctacgcaca tagtcgagct cagcattatt
agccctcctg tatgtttttt ccattaatat atatactttc aagacactag tattcagctt
aaagtgacat ttaaagacta aactaggtta attaggggga aaagtagagt aagtcattgt
ataatagtgg tttgttctgg agacaatcca aaactaatat tgcttaaggg ggctaataaa
attgacctta aaatgaattt aaataattta aaaactgcat ttattctagt cgaaataaaa
gaaataagac tttctttaga agaaaaaaca ttataggaaa tactgcaaaa aaattcctga
atctgttcaa catcattcgg gaaatcaaag gagggctaat aactgtgact tcagctgtac
atcaataaag aggctggttc ttaaattcaa aaaaaaaaaa aaaaaaaaaa aaaaaaaa

The sequence used for the design of the primers of the FSHβ was:

ggtctccacg aaactcccgc agatgaggat gcgtgtgctt gttctggcgc tgctgttgcc
ggtgttaatg agcgcagaat cagaatgcag gtgcagctgt cgactcacca acatctccat
cactgtggag agcgaagaat gtgggagctg cgtcacaatc gacaccacag cctgtgcagg
actatgctgg acaatggatc gagtttaccc tagttccatg gcacagcaca cccagaaggt
ctgtaacttc aagaacttga tgtacaagag ctacgagttt aaaggctgtc ctgcaggggt
tgattcagtc ttcgtgtacc ccgtggctct gagctgtgag tgcaaccagg ttaactcaga
cacaacagac tggggagcta tcagcccgca gaccaccagc tgcagcatac actagagcac
tgtatcatga ccttaacaac atgtacgttg cagaatcaaa ttaagtaagg agtacaatta
gaccatttaa ggatatcaat tatttacaaa acctttagtt tttcatgcat cccacacaca
tggtaatttg gttacttgaa ttaatctgtt gtgttaattc tatagttggt actatggtaa
ctagagtact agagtatcca atgctatact agttttaatt acagttaatt atagaaaagt
atgctacagt atttattaca gtttttctgt tttcaatatt tagtactaca gtatgctagt
gcattcatta acaataagct gtaaatacta taataaatac aggttaatac actttactat
agtatgcttg atcaacacta ttatttaatg tgagttacta tagtactttt caattgggat
ttgtcatttt ggatattgtg ggcttttttg gctattcata aagttttttt tatttttttt
ttatttaatt ttcagtcaaa tggaaacaag tccaccataa tacacttgtg tttcttttgt
caaacttatc aatttgtgtc tgtagatttc aattacaata catattttaa aggccaaaaa
aaaaaaaaaa aaaaaaaa

The sequence used for the design of the primers of the α subunit was: 
gaagacactc atcacgctcc gccggaagtc gaggacaaag ccatcatgtt ttggacaaga
tacgctgaag caagcatttt cttgttgtta atgattcttc atgtcggaca actgtattca
agaaacgatg tgtctaacta tggatgtgaa gagtgcaaac tcaagatgaa cgaacgtttc
 
tccaaacccg gggctccggt ctatcagtgc gtgggctgct gcttttcgag agcttacccc
acacccctga ggtccaagaa aaccatgctt gtcccaaaaa acatcacatc agaagccact
tgctgtgtag caaaagaatc taaaatggtt gccacgaata tcccactata caaccacaca
gactgccact gcagcacctg ttactatcat aagtcttaaa acacactctc ttcacatttc
tcaaatgctc atttcctgtt cttaaatcac agtgactcat gaaatatgat ttttatgtag
ctttccatat ttcaactgtg gccatttcca attcgtttct aaaatggttg gcataagtat
tgtaaactgc atattctgtc actatccctt taagagcgta atatgccatc ctttactatc
attaaatcgc ttatttattt tgttgccttt actgtgacat tcttcaaatc tataaatgaa
ataaaagatt gctgaaggca aaaaaaaaaa aaaaaaaaaa aaaaaaaaaa
 In each case the grey part represents the coding sequence, and the predicted amplified region is underlined.

The primers designed were:

| | |
|---|---|
| Upper-LHβ/EcoRI | 5'-CAA CCG AAT TCA ACG CCT TCA AGA TGT-3' |
| Lower- LHβ/EcoRV | 5'-CCG ATA TCT AGT ATG CGG GGA AAT-3' |
| Upper -FSHβ3 | 5'-AGG ATG CGT GTG CTT GTT CT-3' |
| Lower- FSHβ2/3 | 5'-TGT TGT TAA GGT CAT GAT ACA GTG C-3' |
| Upper-α1 | 5'-GTC GAG GAC AAA GCC ATC AT-3' |
| Lower- α1 | 5'-TGC CAA CCA TTT TAG AAA CGA-3' |

To facilitate further cloning steps the LHβ oligos, include restriction enzyme sites for EcoRI and EcoRV in the upper oligo and in the lower oligo respectively.

Total RNA was isolated from zebrafish heads homogenized in liquid nitrogen and extracted using TRIZOL reagent according to the manufacturer's instructions. Traces of DNA were removed by incubation with DNaseI followed by phenol/chloroform extraction and ethanol precipitation. RT-PCR was performed using the Superscript II one step RT-PCR system with platinum Taq. Reactions were performed with 100ng of total RNA using 25 pmol of the upper and lower primers. Reverse transcription was performed at 50°C for 30 min. PCR conditions were 40 cycles of denaturation at 94°C for 20 s, annealing at and 55°C for LHβ and for FSHβ and 50°C for a during 30 s and extension at 72°C for 1 min followed by a final extension step at 72°C for 10 min. The PCR products were separated by electrophoresis in a 1% gel of agarose and stained with ethidium bromide. The FSHβ PCR product produced a faint band when observed in the agarose gel, in order to optimise we did a PCR using as template this PCR product the reaction was performed with 1/20 of the PCR product reaction using 10µM of the upper an lower primers. PCR conditions were 40 cycles of denaturation at 94°C for 20 s, annealing was performed in a gradient at from 50 °C to 60°C for 30 s and extension at 72°C for 1 min followed by a final extension step at 72°C for 10 min. A sharp band was then observed. To confirm the identity of the amplified sequences, the PCR products were cloned in pCRII-TOPO vector, digested with restriction enzymes to identify the correct direction and sequenced. The analysis of the sequence revealed that we had cloned LHβ, α and FSHβ subunits of zebrafish. These constructs allow now sub-cloning the genes under a constitutive promoter.

Cloning LHβ, FSHβ and α under the control of a constitutive promoter (CMV). The p3XFLAG-CMV-9 expression vector is used to establish transient or stable fusion proteins. The vector encodes three adjacent FLAG epitopes upstream from the multicloning region. This results in an increased detection using anti-FLAG antibody. The promoter-regulatory region of the CMV drives transcription of flag fusion constructs. The preprotrypsin leader sequence precedes the FLAG sequence, promoting the secretion of the protein. The amino glycoside phosphotransferase gene (Neo) confers resistance to amino glycosides such as Geneticin (G418), allowing for selection of stable transfectants.

We used this vector because it has the advantages that the synthesized proteins will be secreted driven by the preprotrypsin leader, we can detect the expression of the proteins by western blots with the anti-FLAG antibody and we can make stable cell lines selecting with geneticin.

### Cloning of LHβ

The PCR product was purified and digested with EcoRI and EcoRV as well as the p3XFLAG-CMV-9 expression vector. The DNA fragments were ligated overnight at 4 °C. The ligation mixture was then used to transform chemical competent cells. Enzymatic digestions selected positive clones. Those clones that give a correct pattern of digestion were sequenced. At the moment, the clone sequenced showed an incorrect insertion of LHβ so I had to repeat this cloning step. The predicted amino acid sequence gives a protein of 187 amino acids with a molecular weight of 20593.8

### Cloning of FSHβ

pCRII-TOPO FSHβ was digested with BamHI/ NotI the band that corresponds to FSHβ cDNA was purified and sub-cloned in the p3XFLAG-CMV-9. The positive clones were then digested then with Not/EcoRV and the mug bean nuclease and religated to get FSHβ in frame with the preprotrypsin leader, these construct was designated as CMV-FSHβ. Alternatively, pCRII-TOPO FSHβ was digested with Xba/BamHI and the resulting band was subcloned in the p3XFLAG-CMV-9. The positive clones were then digested then with EcoRV and religated to get FSHβ in frame with the preprotrypsin leader, this construct was designated as CMV-FSHβ. Enzymatic digestions selected positive clones; those clones that give a correct pattern of digestion were sequenced. The sequencing of the two different colonies, which correspond to the different strategies of cloning, was correct. These constructs were used for the transformation of the ZF4 cell line. The predicted amino acid sequence gives a protein of 174/183 amino acids with a molecular weight of 19062.24/19965.21 respectively to the two different strategies of cloning.

### Cloning of α

pCRII-TOPO α was digested with KpnI/XbaI and the resulting band was subcloned in the p3XFLAG-CMV-9. Enzymatic digestions selected positive clones. Those clones that give a correct pattern of digestion were sequenced. The analysis of the sequence revealed that we have cloned a in the correct orientation and in frame with the flag and the preprotrypsin leader sequence this construct received the name CMV-α. The predicted amino acid sequence gives a protein of 190 amino acids with a molecular weight of 21083.24

The plasmids CMV-FSHβ and CMV-α were purified by alkaline lysis with SDS using the QIAprep spin Miniprep kit. The purified plasmids were linearised with ScaI. The linearised products were separated by electrophoresis in a 1% gel of agarose and stained with ethidium bromide. The linearised plasmid was purified and quantified.

### Transfection of FSHβ in zebrafish fibroblast cell line (ZF4)

The ZF4 (ATCC number: CRL-2050) cells are fibroblast from 1 day-old zebrafish embryos. The frozen aliquot of ZF4 cells was removed from the liquid nitrogen and placed immediately on ice for 10 minutes. The thawed cell suspension is removed from the vial and diluted in 10 ml of complete medium (1:1 mixture of Dubelco's modified Eagle's medium and Ham's F12 containing 1.2g/L of sodium bicarbonate, 2.5mM L-glutamine, 15mM HEPES and 0.5mM sodium pyruvate, 10% of foetal bovine serum and penicillin/streptomycin) at room temperature. The supernatant is discarded by centrifugation at 1200 rpm for 8 min. The cells are resuspended in 8 ml of complete medium and transferred to a tissue flask (T25) and cultured at 28°C. The cells are examined under the inverted microscope to check for cell density. In cultures with a confluence of 80% the medium is removed and washed with 3 ml of PBS to remove cellular debris and serum. Then 0.5 ml of trypsin solution (0.25%) is added and incubated at room temperature until the cells from the monolayer detach from the flask, when a single cell suspension has been obtained 5 ml of complete medium is added to stop trypsinisation. Viable and nonviable cells are counted using a Fuchs-Rosenthal hemocytometer. Cells were seeded in new flasks at a density of 100- 150 cells/mm² (5X105 in each T25 flask). The cells were incubated at 28°C for maximum 4 days before the next passage. Transfection was realized in zebrafish fibroblasts when they were 50-60% confluent using Fugene 6 following manufacturer's instructions. The Fugene6/DNA complex is prepared in a 6:1 ratio of Fugene6: DNA in medium without serum. The culture medium is removed from the cells and replaced with serum free medium. The Fugene6/DNA complex is added drop wisely and mixed. The cells are incubated at 28°C for 5 hours. Then the medium is removed and replaced with complete medium. As a control Zf4 cell were cotransfected with the pEYFP-N1 plasmid and analysed for positive cells under the Leica confocal at 16 and 24 hours after transfection. Different concentrations of DNA were used to establish the optimal concentration for this plasmid giving as a result that 1 µg of DNA in a surface of 21 cm² was the best concentration to obtain a transformation of approximately 30%.

### Detection of the expression of LHβ, FSHβ and α

### Immunohistochemistry

Transfected cells were analysed also by immunohistochemistry to detect the expression of the protein in the cells. The cells were fixated with p-formaldehyde 2% glutaraldehyde 0.1% in PBS during 10 minutes, to eliminate the fixative cells are washed twice with PBS. Then they were permeabilised for 10 minutes with 0.2% of Triton X-100 in PBS. To reduce auto-fluorescence caused by the fixative NaBH4 2 mg/ml in PBS was added during 10 min. Blocking was done with 0.1%BSA-c, 0.02% cold water fish skin gelatin during 30 minutes. Incubation with the anti-FLAG antibody (1:250) was performed overnight at 4°C in 0.1%BSA-c, 0.02% cold water fish skin gelatin. The antibody was removed and washed with 0.1%BSA-c, 0.02% cold water fish skin gelatin twice 10 min each. The secondary antibody (anti-Rabbit Alexa 488) is added in a dilution 1:1000 in 0.1%BSA-c, 0.02% cold-water fish skin gelatin and incubated at room temperature during 60 min. Then is washed three times with 0.1%BSA-c, 0.02% cold water fish skin gelatin and mount in DABCO/Gelvatol. The analysis of the cells in the Leica confocal revealed expression of the protein in vesicles in the transfected cells. This localization is in agreement with the expected site for proteins that are going to be secreted.

### Western Blot

The different constructs were transfected using Fugene 6 in T25 flasks in duplicate, as control cells were not transfected with DNA, transfected with the empty vector and with the positive control CMV- BAP. The proteins were obtained at the third and fifth day after transfection from the supernatant. The supernatant was concentrated using the amicon columns following the manufacturers instructions. FLAG fusion proteins were immunoprecipitated with Anti-FLAG M2 affinity gel, following manufacturers instructions. Proteins samples were diluted 1:4 with sample buffer boiled and kept at -80°C. For setting the conditions of the western blot only the positive (CMV-BAP) and negative (NO DNA) controls were analysed, samples were loaded in a 12% acrilamide gel, run during 60 min at 50 mAmp. The gels were blotted in nitrocellulose membranes. Membrane was blocked with milk 5% overnight at 4°C and immunodetection was realized against anti FLAG antibody at a 1:250 dilution in milk 5% during one hour at room temperature. Immunodetection was revealed with ECL following manufacturer's instructions. The antibody recognized the expressed BAP protein showing a band of the predicted size. With these experiments we show that transient transfected cells are producing the proteins of interest.

### Making stable cell lines

Cells were transfected in 6 well chambers with linearised and purified DNA using Fugene 6. As a control cells were transfected without DNA. After 3 or 5 day after transfection the complete medium was replaced with complete medium with G418 added. The amount of G418 to kill cells that are not expressing the construct varies from cell line to cell line. For the ZF4 cell line the concentrations suggested by the manufacturer are between 0.8 and 1 mg/ml. Cells were treated with 0.8 and 1 mg/ml in quadruplicates, media was changed daily to wash out the dead cells. This was done during 15 days until the plates that contained the cells that did not have DNA were dead and we could not observe any cell in the plate. Then the transfected cells were incubated with the same concentration of G418 for another 5 days. When a confluent monolayer was obtained, the cells were subcultured into a T25 flask and the concentration of G418 was lowered to 0.5mg/ml in complete media. Stable cell lines will be tested with immunohistochemistry and western blot analysis with anti-FLAG antibody to detect the expression of the protein.

### Bioassay

In order to test if the hormones expressed by the transfected cells are active a simple bioassay is going to be performed. Follicular cells cultures from zebrafish respond to pituitary extracts and/or human chorionic gonadotropin (hCG) by upper or down regulating the expression of different genes. hCG (15IU/ml) increases the expression level of activin βA in a time dependent manner. This effect is evident at 40 min of the treatment and reached a maximal level at 2 h, longer treatment (4 h) causes a diminish of the effect. In contrast activin βB is suppressed in the same conditions. When experiments using different concentrations of hCG are performed a dose dependent response is observed. Goldfish pituitary extract also stimulates expression of activin βA and suppresses activin βB in a dose dependent manner. We plan to use this characteristic of the follicular cells in culture to test if the FSHβ and LHβ are active.

### In vitro follicular cell culture

### Isolation of follicular cells

Young zebrafish were purchased from a pet store and maintained without separation of males and females. Females were anaesthetized with 0.01% tricaine methansulfonate solution for 2 minutes or until they were standing still, and decapitated before dissection. The ovaries were then removed and placed in a 10 mm culture dish with L-15 (Gibco). The follicles from 5 females were carefully separated with the aid of insulin needles. The separated follicles were measured with an ocular micrometer in a dissecting microscope and the healthy viotellogenic follicles around 0.45mm were selected, pooled and cultured in T25 flask for 6 days in M199 medium supplemented with 10% foetal bovine serum at 28°C and 5% CO₂. The medium is changed on the third day of the incubation. During the 6-day incubation follicle cells proliferated significantly, increasing the yield of cells for the experiments. Cells are washed and trypsinised at 28°C for 15 min. Thereafter the cells are washed three times with medium M199 through centrifugation at 1000 rpm for 2 min, and then subcultured in 24 plate at a density of 1X105 cells/ml per well for 24 hours in complete M199 before hormone treatment. The amount of cells was not enough for the experiment, so we should start with 20 females to get enough material.

### Hormone treatment

Different concentrations of the supernatant will be used, as a positive control hCG will be used at a 15IU/ml, and carp pituitary extracts will also be included. With the pituitary extract condition we can compare the amount of cells that should be used to observe an effect in the reproduction capacity of the female eel.

### In vivo injection

hCG was dissolved in 0.9% of NaCl solution in a concentration of 20IU/ml. Each fish will receive 50 µl of saline as a negative control, hCG as a positive control and different concentrations of the supernatant or the purified FSHβ and/or LHβ. At 1, 2, 4, 6 and 12 h after injections fish are killed and ovaries removed for RNA extraction

### RNA extraction

At the end of the hormonal treatment total RNA was isolated from zebrafish ovaries or follicular cells, homogenized in liquid nitrogen and extracted using TRIZOL reagent according to the manufacturer's instructions. Traces of DNA were removed by incubation with DNaseI followed by phenol/chloroform extraction and ethanol precipitation. RT-PCR was performed using the Superscript II one step RT-PCR system with platinum Taq. Reactions were performed with 100ng of total RNA using 25 pmol of the upper and lower primers. Reverse transcription was performed at 50°C for 30 min. PCR conditions were 40 cycles of denaturation at 94°C for 20 s, annealing at 56°C during 30 s and extension at 72°C for 1 min followed by a final extension step at 72°C for 10 min. The PCR products were separated by electrophoresis in a 1% gel of agarose and stained with ethidium bromide.

Designed primers to amplify Activin βA, Activin βB and βActin:

| | |
|---|---|
| Upper-Activin A | 5'-TGC TGC AAG CGA CAA TTT TA -3' |
| Lower-Activin A | 5'-CAT TCG TTT CGG ACT CAA G -3' |
| Upper-Activin B | 5'- CAA CTT AGA TGG ACA CGC TG-3' |
| Lower-Activin B | 5'- GTG GAT GTC GAG GTC TTG TC-3' |
| Upper- β Actin | 5'- CCC CTT GTT CAC AAT AAC CT-3' |
| Lower- β Actin | 5'-TCT GTG GCT TTG GGA TTC A-3' |

### Transplant of the stable cell lines in the eel

Stable cell lines will be transplanted to the eel intraperitoneally. Eels will be anaesthetised and a small incision will be made in the belly. The correct amount of cells that produce a constant amount of hormone will be transplanted. As a negative control cells that do not express hormones and only have the empty vector will be transplanted, and as positive control a group of eels will be injected with pituitary extracts.

### Measurement of the maturation process and egg ripening.

To quantify final maturation of female eels there are different characteristics that change along the maturation process, those are a significant increase of the body weight, increase in the eye diameter and morphological changes during the development of the oocytes. Seven morphological stages of final oocyte maturation are observed during pituitary extract treatments. Non-transparent oocytes (stage 0) are still small and fully filled with fat droplets. Final hydration onsets development into stage 1, showing oocytes with increasing transparency and a centred nucleus. In stage 2, the oocytes are fully transparent and fat droplets are clustering and centering. In stage 3, germinal vesicle (GV) migration occurs. In stage 4, the germinal vesicle is found in the periphery and the fat droplets are located on the opposite side. In stage 5, the fat droplets decrease due to the fat fusion, giving as a result larger fat droplets. At stage 6 meiosis II is completed with disappearance of the germinal vesicle and small numbers of large fat droplets are observed. In stage 7 the oocytes have a single fat droplet. There is a change in the diameter of the oocytes during the first two stages; this is because of the process of hydratation. These morphological changes in the oocyte maturation will allow us to determine the effect of the cell transplantation. After transplantation of the cells, eels will be weighted, and the eye diameter will be measured regularly. During the treatment oocyte samples will be taken in order to determine if the oocytes are responding to the treatment.

### Implants with hormone producing cells

### Introduction

A test was carried out to compare the effectiveness of implantation with hormone producing ZF4 cells with the standard technique of injection with CPE (carp pituitary extract). Sexual maturation can be obtained by repeated injection with CPE as has recently been described by Palstra et al (2005). The repeated injections do not result in quality eggs and viable offspring, likely due to induced stress. Thus the CPE injection protocol has to be replaced by a less stressful procedure, such as implants with hormone producing cells, as described in this example. As the effect of CPE in eel can be observed after a few weeks by morphological, histological, and endocrinological changes, a 4 week test was carried out with female silver eels. The amount of injected cells was related to the estimated weight of the pituitary of the experimental eel.(ca 6 µl tissue / kg eel).

### Materials and Methods

### Hormone producing cells

Three different stable cell lines were grown independently: genes for FSHβ, LHB, and FSH/LHα were inserted in the CMV-promotor and stable transfected in ZF4 cells. According to the protocol as described above (transfected with Fugene 6 and selected with G418).

### GFP-β-galactosidase stable cell line

The pMP2838 plasmid was used to make a ZF4 stable cell line that expresses a fusion protein between the green fluorescent protein (GFP) and the β-galactosidase protein. This plasmid was described by Bakkers (2000). Briefly: the gfpN-LacZ gene of the pUAS-gfpN_LacZ plasmid was taken out and used as replacement for the gfp gene in the pEGFP-C3 plasmid. So, a green fluorescent fusion protein with β-galactosidase activity (gfpN-LacZ) was expressed under control of the CMV promoter. The pMP2838 plasmid also contains the neomycin resistant (Neor) gene that allows the selection of positive clones with gentamicin (G418) as described above for making stable cell lines. In the same way ZF4 stable cell lines were generated with the same protocol as described above (transfected with Fugene 6 and selected with G418).

At the day of the injection cells were harvested and quantified to a total of 10⁸ cells for each cell line. The 4 cell lines were mixed to equal cell concentrations and diluted in DMEM-F12 medium without serum to a final concentration of 20 million cells in one ml.

### β-galactosidase tissue staining

The tissue is briefly rinsed in PBS (phosphate buffered saline) and fixed immediately with paraformaldehyde 1%- glutaraldeyde 0.1% in PBS with MgCl₂ 2mM, EDTA 5mM and NP-40 0.02% during 30 minutes at room temperature. Then it is washed twice during 5 minutes with wash solution (PBS with MgCl₂ 2mM, EDTA 5mM, NP-40 0.02% and Na deoxycholate 0.01%) at room temperature. Thereafter the tissue is stained during 12 hours with stain solution (PBS with MgCl₂ 2mM, NP-40 0.02%,Na-deoxycholate 0.01%, K₃Fe(CN)₆ 5mM K₄Fe(CN)₆ 5mM and X-gal 1mg/ml) at 37°C. The stained tissue is then washed with PBS and embedded in paraffin.

### Animals and Protocol

Sixty female silver eels (900-1600g) were caught in the wild during their seaward migration in Lake Grevelingen (Netherlands). All eels were upon arrival equipped with a microchip (Trovan) for identification, and external parameters were measured. Ten eels were immediately sacrificed as control animals. The remaining female eels were kept in a recirculation system of 3000 liters in artificial seawater (35 promille) at a temperature of 18°C. To prevent bacterial infections at the day of handling, the animals were exposed for 3 hours to the antibiotic Flumequine (50mg/L) in a large separate tank. One group of 24 eels was injected weekly with Carp Pituitary Extract (CPE: 20-mg/kg) according to method described before (Palstra et al 2005), the other group was injected only once at the start of the experiment with 1ml of a mix of 4 types of cells (6-gal, LHβ, FSHβ, LH/FSHα). The cells were injected as a suspension subcutaneously below the beginning of the dorsal fin and above the lateral line.

Each week 6 eels from each group were sacrificed to analyse the treatment effects. These included: total weight, eye index, gonad weight, and pectoral fin length. Furthermore tissue samples (blood, pituitary, liver, gonad) were taken for later analysis. Additionally samples from the places of injection were obtained to test the presence of 6-galactosidase positives cells. From the same eel control samples were obtained from regions where no cells were injected.

### Results & Discussion

### Cell implants

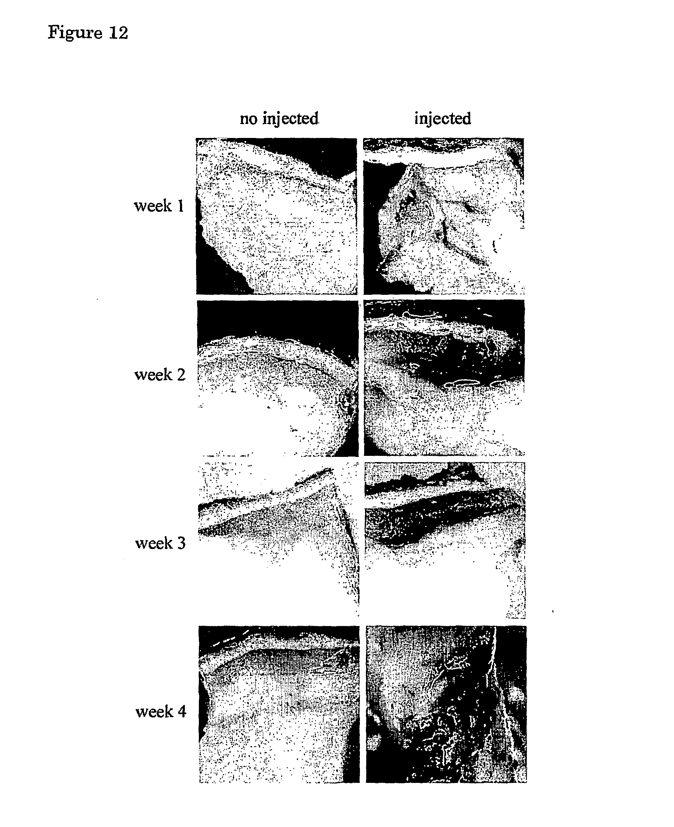

The histological analysis of the subcutaneous implants were checked for 6-galactosidase staining. All samples showed the occurrence of the cells (figure 12). The coloring occurred initially in the fat/collagen tissue that is typical for the subcutane layers, however, also in many cases infiltration around the muscle layers could be observed. Comparing the samples between week 1 and week 4, there was often even an increase of the total mass of 6-galactosidase staining cells, clearly indicating that those cells are capable to infiltrate the host tissue. There was no indication for rejection, this is a remarkable observation since ZF4 cells originate from zebrafish, which is evolutionary very distant from eel.

### Morphological changes

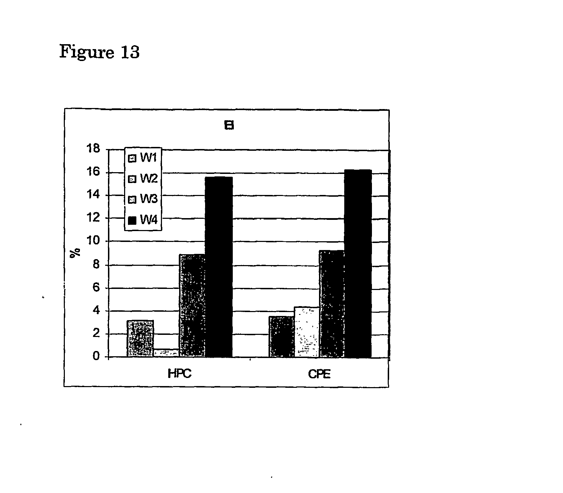

The most prominent and proximate change that indicates the onset of maturation is the increase of the eye size in silver eels (Durif et al 2004). From figure 13 it is clear that the observed changes in eye index - [surface area relative to length, Pankhurst 1982] - induced by the cell implants are almost identical to those induced by CPE injection. This result clearly shows the effectiveness of cell implants. Further proof is to be obtained from a 3-4 month stimulation of silver eels, which should result in final maturation.

### Conclusions

At least two conclusions can be drawn from the above experiment: 1) zebrafish ZF4 cells (fibroblast cell line) are not rejected by silver eels over a period of 4 weeks, and are even found to proliferate. 2) The implanted mix of cells had a similar effect on the eye index as did the standard protocol with CPE, thus showing evidence for hormonal stimulation.

**Using the protocol and constructs described above** Eel cell line are generated and transplanted into eels. Further fish cells expressing both the units (α+β) are generated.

### References:

Palstra, A.P., Cohen, E.G.H., Niemantsverdriet, P.R.W., van Ginneken, V.J.T., van den Thillart, G.E.E.J.M. (2005) Artificial maturation and reproduction of European silver eel: Development of oocytes during final maturation. Aquaculture, 2005.
Durif, C., Dufour, S., Elie, P. (2005) The silvering process of the eel : a new classification from the yellow resident stage to the silver migrating stage. J. Fish Biol. 66, 1-19
Pankhurst, W.N. (1982) The relation of visual changes to the onset of sexual maturation in European eel, Anguilla anguilla L. J. Fish Biol. 21, 179-196
Bakker J. (2000) Chitin oligosacharides in Zebrafish development. Investigations at the molecular and cellular level. (Thesis University of Leiden).

### Brief description of the drawings and figures

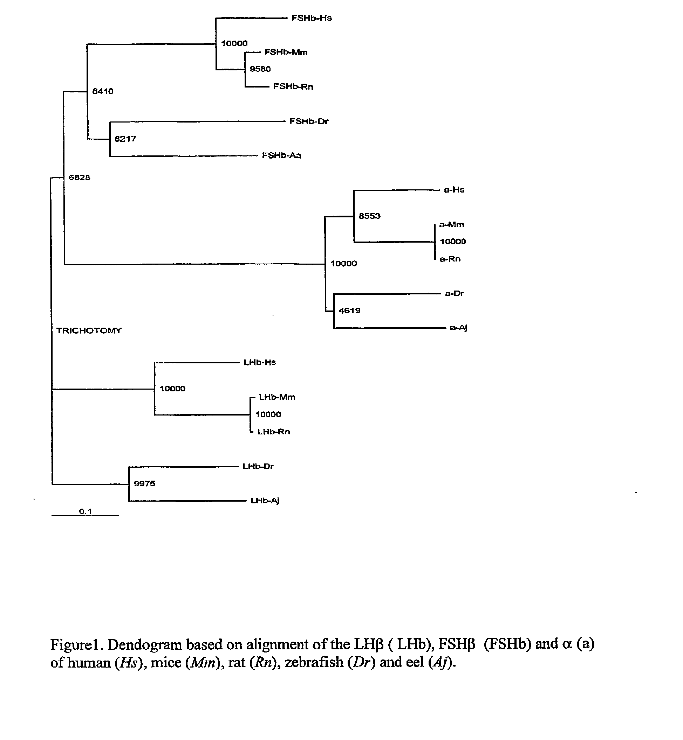

Figure1. Dendrogram based on alignment of the LHβ (LHβ), FSHβ (FSHβ) originating from a (a) of human (Hs), mice (Mm), rat (Rn), zebrafish (Dr) and eel (Aj).

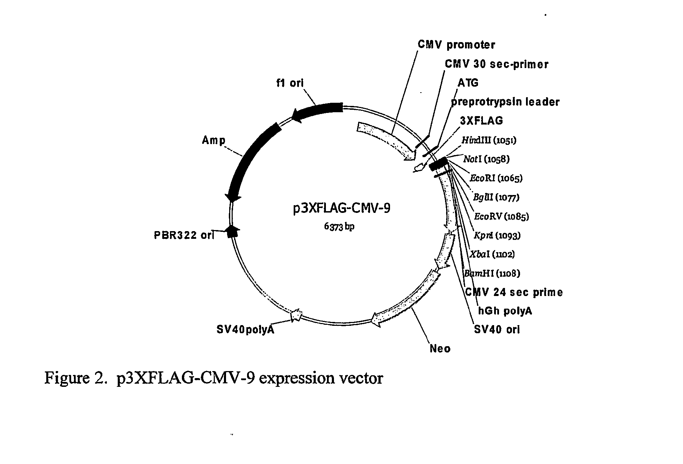

Figure 2. p3XFLAG-CMV-9 expression vector

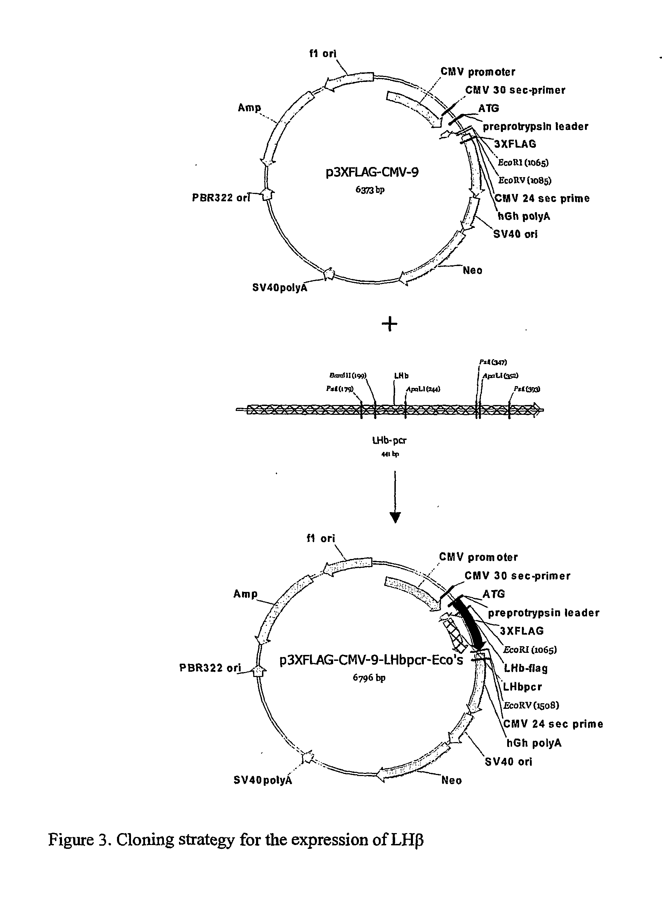

Figure 3. Cloning strategy for the expression of LHβ

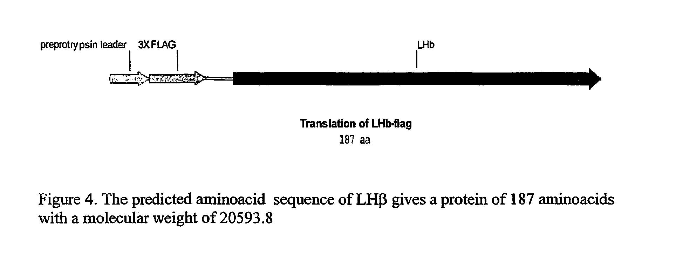

Figure 4. The predicted aminoacid sequence of LHb gives a protein of 187 aminoacids with a molecular weight of 20593.8

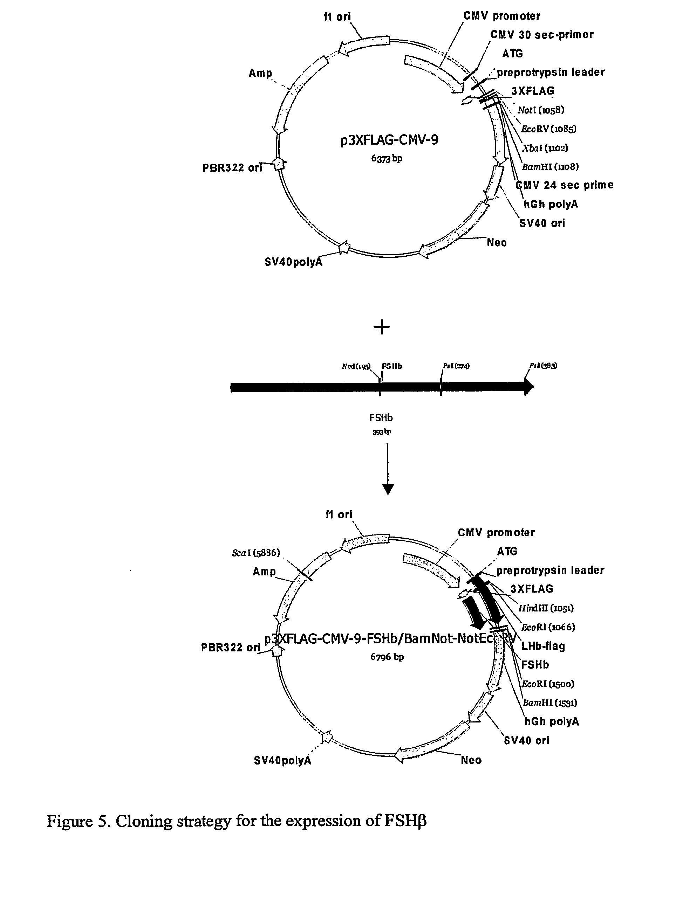

Figure 5. Cloning strategy for the expression of FSHβ

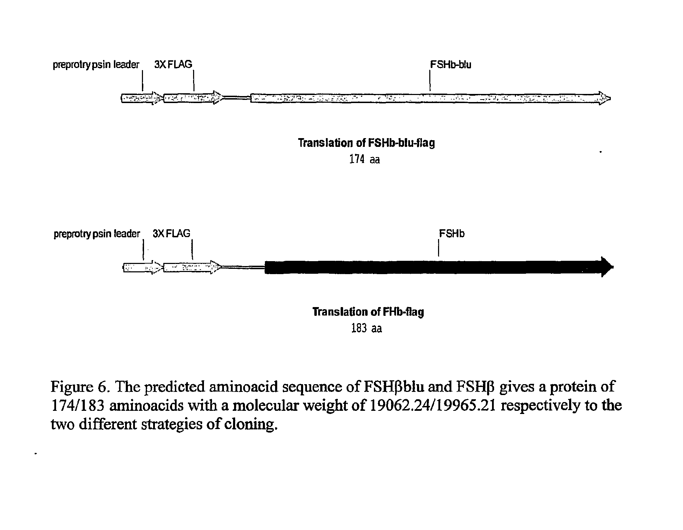

Figure 6. The predicted aminoacid sequence of FSHbblu and FSHb gives a protein of 174/183 aminoacids with a molecular weight of 19062.24/19965.21 respectively to the two different strategies of cloning.

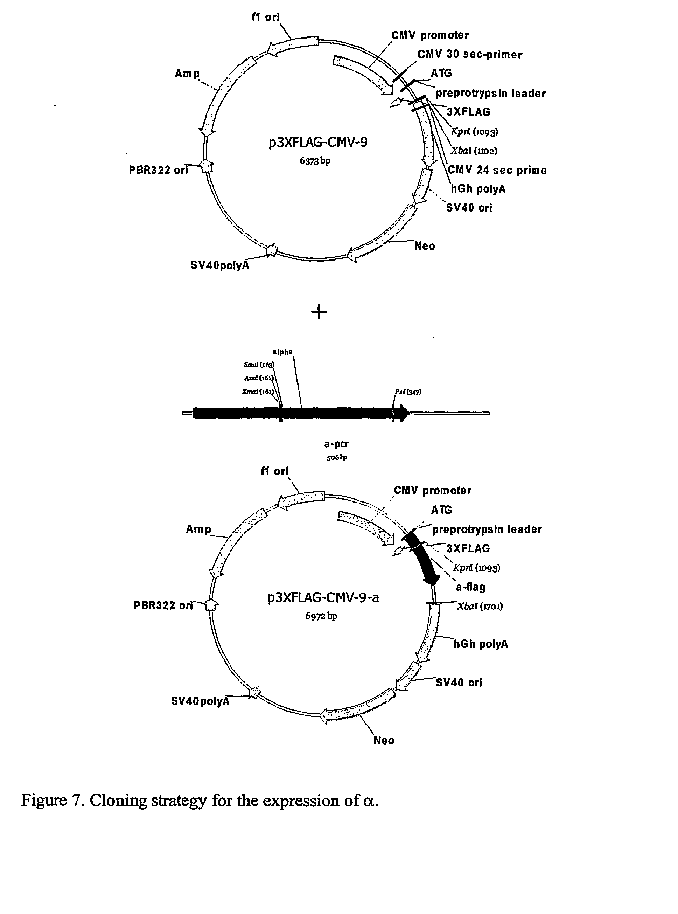

Figure 7. Cloning strategy for the expression of α.

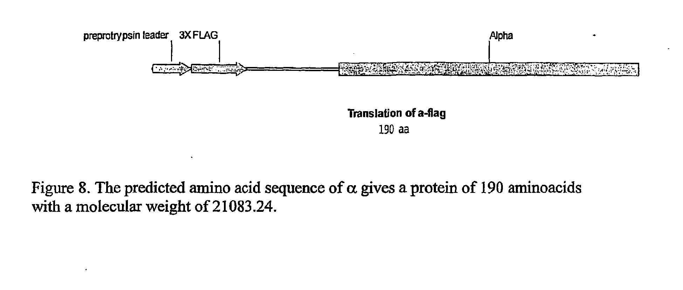

Figure 8. The predicted amino acid sequence gives a protein of 190 amino acids with a molecular weight of 21083.24.

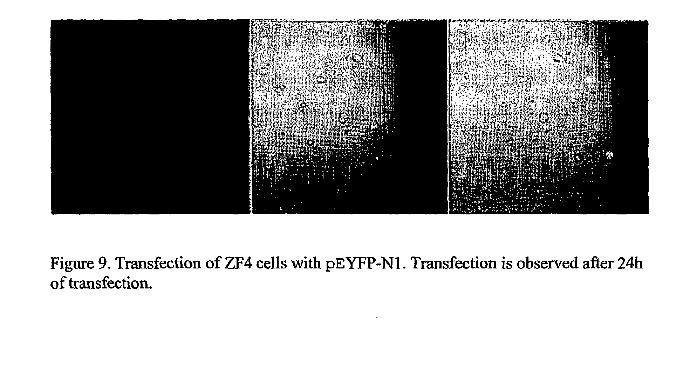

Figure 9. Transfection of ZF4 cells with pEYFP-N1. Transfection is observed after 24h of transfection.

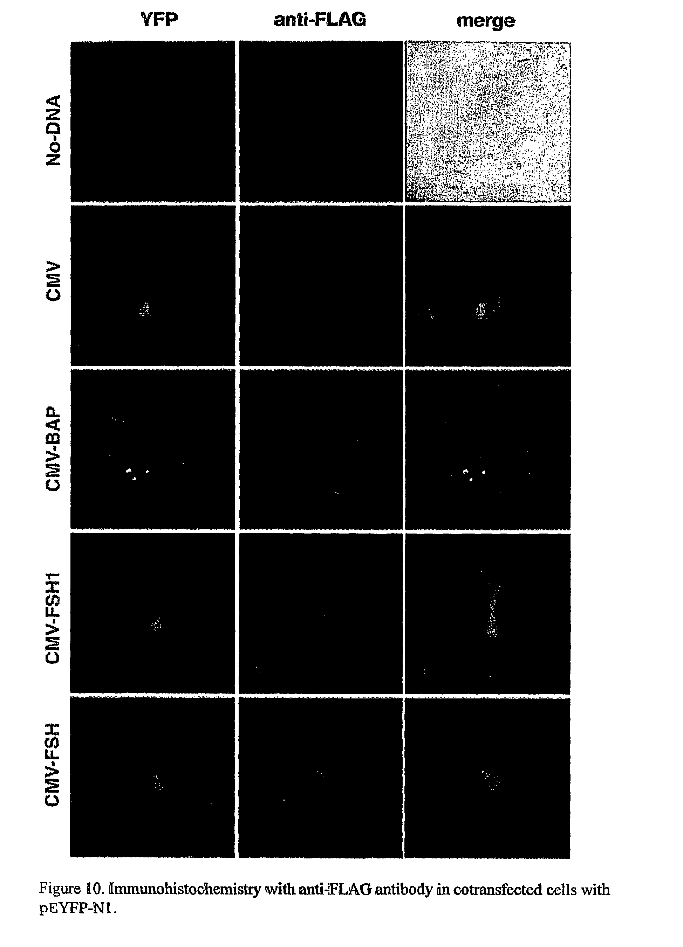

Figure 10. Immunohistochemistry with anti-FLAG antibody in cotransfected cells with pEYFP-N1.

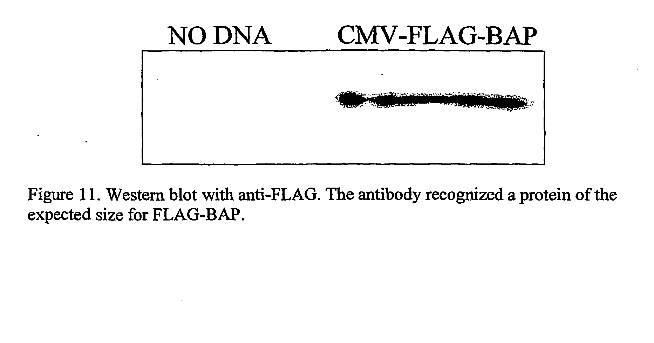

Figure 11. Western blot with anti-FLAG. The antibody recognized a protein of the expected size for FLAG-BAP.
Figure 12. Staining for 6-galactose indicated the occurrence of β-gal-ZF4 cells that were injected subcutaneously in silver eels. From the same eel skin/muscle tissue was dissected from the injected area and an area farther towards the tail. Subcutaneous fat tissue is easily recognisable in the figures, as is the dermis and the muscle layer.
Figure 13. Eye Index (EI) changes in regard to the initial stage (%) of silver eels during 4 weeks (W1-W4) of treatment with hormone producing cell (HPC) or with carp pituitary extracts (CPE). For the initial stage and each of the weeks 6 full-grown female silver eels were taken (weight 1300 ± 300g).

## Claims

1. A method for improving the development and/or maturation of eggs and/or sperm in fish using fertility hormone administration comprising providing said fish with cells producing said fertility hormone for use in the creation of progeny or the production of eggs.

2. A method according to claim 1, wherein said fertility hormone comprises luteinizing hormone (LH), follicle stimulating hormone (FSH)or chorionic gonadotropin (CG) or a functional part thereof.

3. A method according to claim 1 or claim 2, wherein said cells are genetically modified to express said fertility hormone.

4. A method according to any one of claims 1-3, wherein said fertility hormone is derived from the same genus as said fish.

5. A method according to any one of claims 1-4, wherein said cells have been provided with one or more genes encoding said fertility hormone.

6. A method according to any one of claims 1-5, wherein said cells are a clonal population.

7. A method according to any one of claims 1-6, wherein said cells have been selected for a reduced immunogenicity in said fish.

8. A method according to any one of claims 1-7, wherein said fish are diadromous fish.

9. An isolated and/or recombinant culturable fish cell that produces a fertility hormone.

10. A fish cell according to claim 9, wherein said cell is genetically modified to express said fertility hormone.

11. A fish cell according to claim 9 or claim 10, wherein said cell is an eel cell.

12. A fish that comprises a cell according to any one of claims 9-11.

13. A fish according to claim 12, that is a consumer fish.

14. A fish according to claim 13 that is an eel.

15. A fish according to claim 14, wherein said eel belongs to the genus Anguilla.

## Patentansprüche

1. Verfahren zur Verbesserung der Entwicklung und/oder Reifung von Eiern und/oder Sperma bei Fischen mittels Verabreichung eines Fruchtbarkeitshormons, umfassend das Verstehen der Fische mit Zellen, die das Fruchtbarkeitshormon produzieren, zur Verwendung beim Hervorbringen von Nachkommen oder der Produktion von Eiern.

2. Verfahren gemäß Anspruch 1, wobei das Fruchtbarkeitshormon luteinisierendes Hormon (LH), follikelstimulierendes Hormon (FSH) oder Choriogonadotropin (CG) oder einen funktionellen Teil davon umfasst.

3. Verfahren gemäß Anspruch 1 oder 2, wobei die Zellen genetisch so modifiziert sind, dass sie das Fruchtbarkeitshormon exprimieren.

4. Verfahren gemäß einem der Ansprüche 1 bis 3, wobei das Fruchtbarkeitshormon von derselben Gattung stammt wie der Fisch.

5. Verfahren gemäß einem der Ansprüche 1 bis 4, wobei die Zellen mit einem oder mehreren Genen, die das Fruchtbarkeitshormon codieren, versehen sind.

6. Verfahren gemäß einem der Ansprüche 1 bis 5, wobei es sich bei den Zellen um eine klonale Population handelt.

7. Verfahren gemäß einem der Ansprüche 1 bis 6, wobei die Zellen so selektiert sind, dass sie eine reduzierte Immunogenität in den Fischen aufweisen.

8. Verfahren gemäß einem der Ansprüche 1 bis 7, wobei die Fische Wanderfische sind.

9. Isolierte und/oder rekombinante kultivierbare Fischzelle, die ein Fruchtbarkeitshormon produziert.

10. Fischzelle gemäß Anspruch 9, wobei die Zelle genetisch so modifiziert ist, dass sie das Fruchtbarkeitshormon exprimiert.

11. Fischzelle gemäß Anspruch 9 oder 10, wobei die Zelle eine Aalzelle ist.

12. Fisch, der eine Fischzelle gemäß einem der Ansprüche 9-11 umfasst.

13. Fisch gemäß Anspruch 12, bei dem es sich um einen zum menschlichen Verzehr bestimmten Fisch handelt.

14. Fisch gemäß Anspruch 13, bei dem es sich um einen Aal handelt.

15. Fisch gemäß Anspruch 14, wobei der Aal zur Gattung *Anguilla* gehört.

## Revendications

1. Un procédé pour améliorer le développement et/ou la maturation des oeufs et/ou du sperme chez le poisson par administration d'une hormone de fertilité comprenant la fourniture audit poisson de cellules produisant ladite hormone de fertilité pour une utilisation dans la création de progéniture ou la production d'oeufs.

2. Un procédé selon la revendication 1, dans lequel ladite hormone de fertilité comprend lutéïnisante (LH), l'hormone stimulante du follicule (FSH) ou la gonadotrophine chorionique (CG) ou une partie fonctionnelle en dérivant.

3. Un procédé selon la revendication 1 ou revendication 2, dans lequel lesdites cellules sont génétiquement modifiées pour exprimer ladite hormone de fertilité.

4. Un procédé selon l'une quelconque des revendications 1 à 3, dans lequel ladite hormone de fertilité provient du même genre que ledit poisson.

5. Un procédé selon l'une quelconque des revendications 1 à 4, dans lequel lesdites cellules ont été pourvues de un ou plusieurs gènes codant pour ladite hormone de fertilité.

6. Un procédé selon l'une quelconque des revendications 1 à 5, dans lequel lesdites cellules sont une population clonale.

7. Un procédé selon l'une quelconque des revendications 1 à 6, dans lequel lesdites cellules ont été sélectionnées pour une immunogénicité réduite chez ledit poisson.

8. Un procédé selon l'une quelconque des revendications 1 à 7, dans lequel lesdits poissons sont des poissons diadromes.

9. Une cellule de poisson cultivable isolée et/ou recombinante qui produit une hormone de fertilité.

10. Une cellule de poisson selon la revendication 9, dans laquelle ladite cellule est génétiquement modifiée pour exprimer ladite hormone de fertilité.

11. Une cellule de poisson selon la revendication 9 ou la revendication 10, dans laquelle ladite cellule est une cellule d'anguille.

12. Un poisson qui comprend une cellule selon l'une quelconque des revendications 9 à 11.

13. Un poisson selon la revendication 12, qui est un poisson consommable.

14. Un poisson selon la revendication 13, qui est une anguille.

15. Un poisson selon la revendication 14, dans lequel ladite anguille appartient au genre Anguilla.
